# EUROPEAN PATENT SPECIFICATION

(11) **EP 0 617 965 B1**
(45) Date of publication and mention of the grant of the patent: **15.11.2000**
(21) Application number: 94200650.3
(22) Date of filing: 14.03.1994
(51) Int. Cl.: A61K 38/02

(54) **Pharmaceutical preparation based on Clara cell protein CC10, and it's use as medicament and diagnosticum**
Clarazell-Protein CC10 enthaltendes pharmazeutisches Präparat und seine Verwendung als Arzneimittel und Diagnostikum
Préparation pharmaceutique à base de la protéine CC10 de cellules clara et son application comme médicament et en diagnostique

(30) Priority: 15.03.1993 BE 9300242
(43) Date of publication of application: 05.10.1994
(73) Proprietor: De Ley, Marc Jozef Philemon, B-3001 Leuven-Heverlee (BE)
(72) Inventor: De Ley, Marc Jozef Philemon, B-3001 Leuven-Heverlee (BE)
(74) Representative: Prins, Hendrik Willem

(56) References cited:
- WO-A-89/05147
- CLINICA CHIMICA ACTA vol. 207, no. 3 , 15 May 1992 , AMSTERDAM, NL pages 239 - 249 A. BERNARD ET AL. 'HUMAN URINARY PROTEIN 1: EVIDENCE FOR IDENTITY WITH THE CLARA CELL PROTEIN AND OCCURRENCE IN RESPIRATORY TRACT AND UROGENITAL SECRETIONS.'
- BIOCHEMICAL AND BIOPHYSICAL RESEARCH COMMUNICATIONS vol. 152, no. 3 , 16 May 1988 , DULUTH, MINNESOTA US pages 1447 - 1454 R. DHANIREDDY ET AL. 'DETECTION OF A RABBIT UTEROGLOBIN-LIKE PROTEIN IN HUMAN NEONATAL TRACHEOBRONCHIAL WASHINGS.'
- CHEMICAL ABSTRACTS, vol. 113, no. 17, 22 October 1990, Columbus, Ohio, US; abstract no. 147437, G. SINGH ET AL. 'CLARA CELL 10 kDa PROTEIN (CC10): COMPARISON OF STRUCTURE AND FUNCTION TO UTEROGLOBIN.' page 293 ; & BIOCHIM. BIOPHYS. ACTA vol. 1039, no. 3 , 1990 pages 348 - 355

## Description

The present invention relates to a pharmaceutical preparation based on Clara cell protein CC10 and to the use of Clara cell protein CC10 as a medicament and diagnosticum.

Clara cell protein CC10, also called urinary protein 1, or simply CC10 or CC16, is known from the literature (Singh G. et al., Biochimica Biophysica Acta 950 [1988] 329-337). Clara cell protein CC10 is a homodimeric disulphide-linked polypeptide (Mᵣ 2 x 7900) which occurs in several biological fluids, such as sputum, urine, semen and in the mucous membranes of the airways.

In sputum the Clara cell protein CC10 concentration amounts to an average 15 mg/l. The concentration in urine is greater in males (30 µg/l) than in females (0.5 µg/l), these differences becoming maximal at puberty. In addition, the concentration of Clara cell protein CC10 in the urine rises during pregnancy.

Little is known about the biological function of Clara cell protein CC10 at this moment. CA 147437h, vol.113, page 293,(1990) reports the exacerbation of carragenan-induced foot pad swelling.

The present invention is based on the discovery that Clara cell protein CC10 inhibits the production of interferon-gamma (= IFN-gamma) of activated mononuclear cells. This inhibition is maximal when interleucine 2 (IL-2) is used for stimulation. Clara cell protein CC10 inhibits on the one hand the IL-2 production but in particular blocks the interaction of IL-2 with target cells or a combination of both.

It has been found from this discovery, that Clara cell protein CC10 acts as an immuno-suppressive agent as a result of its anti-cytokine action.

A pharmaceutical preparation contains Clara cell protein CC10 or an active derivative thereof as active constituent, and a pharmaceutically acceptable carrier or diluent. This pharmaceutical preparation can be used in the treatment of organisms, in particular humans and animals, as a medicine or diagnostic.

The pharmaceutical preparation can contain the Clara cell protein CC10 in combination with any pharmaceutically acceptable carrier or diluent.

The dosage forms can consist of tablets, granules, inhalable powder, aerosols, parenterally, intramuscularly, intravenously administrable fluids, and such like. Particular preference is given to injectable fluids which are isotone and optionally buffered in usual manner.

The pharmaceutical preparation can be administered in a suitable dosage form in a quantity dependent on the concentration of the active constituent of Clara cell protein CC10. This quantity generally amounts to less than 10 mg/kg body weight, generally less than 5 mg/kg, for instance within the range of a maximum of 0.05 to 5 mg/kg body weight. This dosage may be administered all at once or in multiple doses spread over the day, depending on the type of disease, the nature of the disease, age and gender of the patient.

As a result of the anti-cytokine action of the Clara cell protein CC10, Clara cell protein CC10 finds particular application in the treatment of auto-immune diseases, such as thyroiditis Hashimoto, lupus erythematosus, rheumatoid arthritis, multiple sclerosis, chronic infection.

Clara cell protein CC10 can further be applied for use in preventing tissue rejection after transplantation; for treatment of infertility, septic shock, allergy, biliary cirrhosis, myasthenia gravis; or to prevent premature birth, abortion by Gram-negative septicaemia.

Clara cell protein CC10 can moreover be used as diagnostic, because the concentration thereof in body fluids such as urine, blood, amniotic fluid, synovial fluid, lung-lavage, cerebrospinal fluid, seminal fluid and the like represents a measure of the immune status and gives an impression of the operation of the so-called cytokine-network, whereby the concentration of the Clara cell protein has a predictive value in respect of the above mentioned diseases.

It will be apparent to the skilled person that in addition to Clara cell protein CC10, derivative protein and polypeptide structures are also suitable for use according to the invention, insofar as these structures display substantially the same biological activity.

The anti-cytokine action of Clara cell protein CC10 will be elucidated hereinafter on the basis of a number of examples which clearly show the immune mediating action at the level of a blocking of the IFN-gamma production, particularly when induced by IL-2.

### EXAMPLE 1

Mononuclear cells (MNC) originating from a single donor were isolated from fresh buffy coats by centrifugation on lymphoprep (Nycomed Pharma AS, Oslo, Norway) and cultured in RPMI 1640 medium (Gibco Ltd., Paisley, Scotland) supplemented with 10%(v/v) heat-inactivated foetal calf serum with 0.1%(v/v) gentamicin and L-glutamine (2mM). Where necessary monocytes were removed by adhesion to plastic (1 hour, 37°C, 5%(v/v) CO₂), whereafter the lymphocyte purity was assessed by a non-specific esterase staining. The cells were incubated in the presence of Clara cell protein CC10 and stimulated after 3 hours by adding an inducing agent. Samples were taken from the supernatant every 24 hours for 4-7 days.

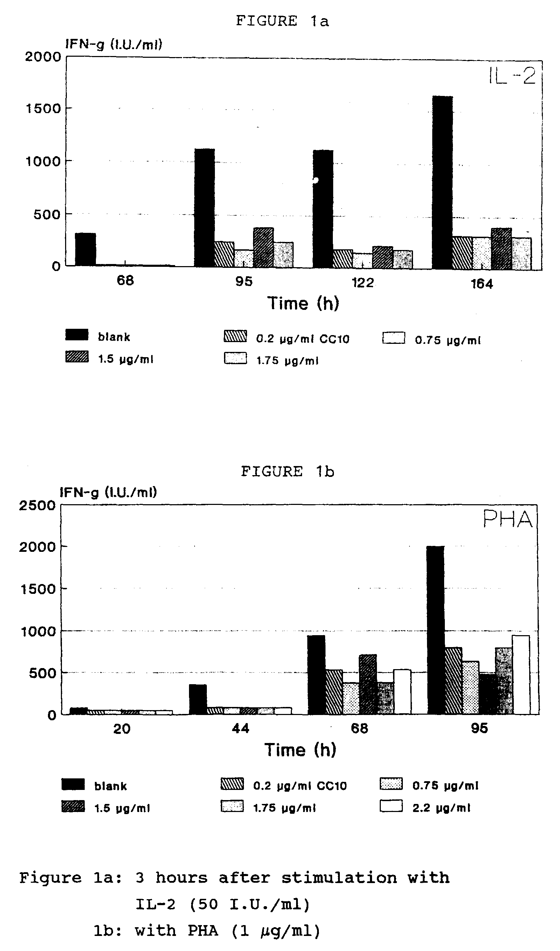

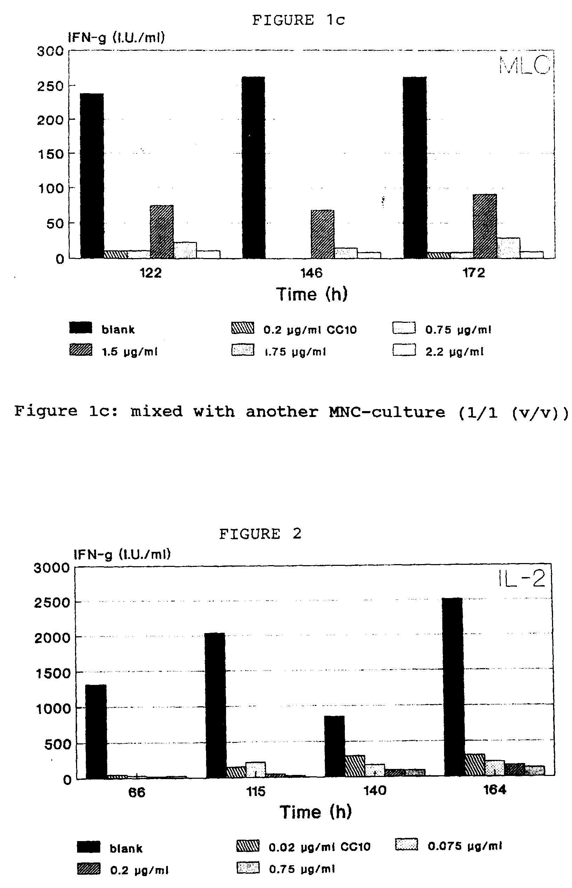

Figure 1 shows the IFN-gamma production by these mononuclear cells ( 2 x 10⁶ cells/ml) after stimulation with phytohemagglutinin (PHA, Welcon, Beckingham, UK), IL-2 and a mixed leucocyte culture (MLC). As can be seen from figure 1 the production of IFN-gamma is clearly inhibited. The IFN-gamma production was minimal in the presence of 1.5 µg/ml Clara cell protein CC10 when IL-2 or MLC were used. The extent of maximum inhibition varied from 75% (PHA) to 95% (IL-2 and MLC).

The IFN-gamma concentration was measured using a standard Elisa test and the obtained values were corrected with respect to the international standard preparation Gg 23-901-530-g (National Institute of Allergy and Infectious Diseases, NIH, Bethesda, USA).

The IFN-gamma concentration was further assayed in an anti-viral test making use of human A459 cells, which were infected with encephalomyocarditis virus (EMC virus) after a 24-hour period of incubation with dilutions of the supernatant. Once again the values were corrected against an international standard (National Biological Standards Board, National Institute of Standards and Control, Hertfordshire EN6 3QG UK, Human IL-2 Standard 86-504).

### EXAMPLE 2

MNC depleted of monocytes by adhesion to plastic (lymphocyte purity of 95%) were stimulated with IL-2. As shown in figure 2, there was only a slight increase in the total production of IFN-gamma. Conversely, the extent of inhibition induced by including Clara cell protein CC10 in a culture medium remained constant. The inhibition by Clara cell protein CC10 is therefore not the result of inactivation of the monocyte function.

### EXAMPLE 3

Clara cell protein CC10 is radioactively labelled with [¹²⁵I] according to the chloramine-T-method. The [¹²⁵I]-CC10 was subsequently incubated with 3 x 10⁶ MNC or A549 cells in the same medium as in example 1 for 2 hours at 4°C. The same experiment was repeated in the presence of an excess of non-radioactively labelled CC10. After separation CC10 is found to have adhered to the MNC or A549 cells for less than 2%.

### EXAMPLE 4

Example 1 was repeated with CC10 monomer, i.e. after reduction of the disulphide bridges with dithiothreitol and blocking of the thiol groups by means of pyridyl ethylation. The effect was verified of CC10 monomer on the interferon-gamma production by OK-432 stimulated MNC cells. In contrast to dimer CC10, which inhibited the IFN-gamma production by 33% at a concentration of 100 ng/ml, no effect of the CC10 monomer was observed at the same concentration.

### EXAMPLE 5

Clara cell protein CC10 reduces the phagocytosis of monocytes as a result of stimulation with interferon-gamma. The phagocytosis by monocytes, which is generated by addition of zymosan A (trademark), was measured by means of bioluminescence after overnight pre-incubation with 10 units/ml interferon-gamma, which resulted in an increase of 68%. If prior to the incubation with interferon-gamma the cells were treated for 3 hours with Clara cell protein CC10 (concentration 100 ng/ml), the effect of interferon-gamma was inhibited by more than 70%.

### EXAMPLE 6

Human leukocytes were stimulated to interferon-gamma production, with or without pretreatment with Clara cell protein CC10. Subsequently the RNA was extracted, and the mRNA, which codes for interferon-gamma, was then quantified. By means of PCR it was demonstrated that a significant decrease in the quantity of mRNA occurred as a result of the treatment with Clara cell protein CC10.

## Claims

1. Clara cell protein CC10 or an active derivative thereof for use as medicine.

2. Clara cell protein CC10 or an active derivative thereof, as claimed in claim 1, for use as inhibitor of cytokine action.

3. Clara cell protein CC10 or an active derivative thereof, as claimed in claim 1, for use in the treatment of an auto-immune disease.

4. Clara cell protein CC10 or an active derivative thereof, as claimed in claim 1, for use in the treament of thyroiditis Hashimoto, lupus erythematosus, rheumatoid arthritis, multiple sclerosis, chronic infection.

5. Clara cell protein CC10 or an active derivative thereof, as claimed in claim 1, for use:
i) in preventing tissue rejection after transplantation;
ii) for treatment of infertility, septic shock, allergy, biliary cirrhosis, myasthenia gravis; or
iii) to prevent premature birth, abortion by Gram-negative septicaemia.

6. Clara cell protein CC10 or an active derivative thereof, as claimed in claim 1, for use as diagnostic.

7. Clara cell protein CC10 or an active derivative thereof, as claimed in claim 1, for use in determining the immune status of the organism.

## Patentansprüche

1. Clara-Zellprotein CC10 oder ein aktives Derivat hiervon zur Verwendung als Arzneimittel.

2. Clara-Zellprotein CC10 oder ein aktives Derivat hiervon nach Anspruch 1 zur Verwendung als Inhibitor der Cytokin-Wirkung.

3. Clara-Zellprotein CC10 oder ein aktives Derivat hiervon nach Anspruch 1 zur Verwendung bei der Behandlung einer Auto-Immun-Erkrankung.

4. Clara-Zellprotein CC10 oder ein aktives Derivat hiervon nach Anspruch 1 zur Verwendung bei der Behandlung von Thyroiditis Hashimoto, Lupus Erythematosus, rheumatoider Arthritis, multipler Sklerose, chronischer Infektion.

5. Clara-Zellprotein CC10 oder ein aktives Derivat hiervon nach Anspruch 1 zur Verwendung:
i) bei der Vorbeugung einer Gewebsabstoßung nach Transplantation;
ii) zur Behandlung der Unfruchtbarkeit, des septischen Schocks, Allergie, biliärer Zirrhose, Myasthenia Gravis; oder
iii) zur Vorbeugung einer Frühgeburt, Schwangerschaftsabbruch durch gram-negative Septicaemie.

6. Clara-Zellprotein CC10 oder ein aktives Derivat hiervon nach Anspruch 1 zur Verwendung als Diagnostikum.

7. Clara-Zellprotein CC10 oder ein aktives Derivat hiervon nach Anspruch 1 zur Verwendung bei Bestimmung des Immun-Status des Organismus.

## Revendications

1. Protéine CC10 de cellules Clara ou dérivé actif de celle-ci destiné à être utilisé en tant que médicament.

2. Protéine CC10 de cellules Clara ou dérivé actif de celle-ci, selon la revendication 1, destiné à être utilisé en tant qu'inhibiteur de l'action des cytokines.

3. Protéine CC10 de cellules Clara ou dérivé actif de celle-ci, selon la revendication 1, destiné à être utilisé dans le traitement d'une maladie auto-immune.

4. Protéine CC10 de cellules Clara ou dérivé actif de celle-ci, selon la revendication 1, destiné à être utilisé dans le traitement de la thyroïdite de Hashimoto, du lupus érythémateux, de la polyarthrite rhumatoïde, de la sclérose en plaque, et d'une infection chronique.

5. Protéine CC10 des cellules Clara ou dérivé actif de celle-ci, selon la revendication 1, destiné à être utilisé :
i) dans la prévention du rejet de tissu aprés transplantation ;
ii) pour le traitement de la stérilité, du choc septique, de l'allergie, de la cirrhose biliaire, de la myasthénie gravis ; ou
iii) pour éviter les accouchements prématurés et les fausses couches dus à une septicémie gram-négative.

6. Protéine CC10 des cellules Clara ou dérivé actif de celle-ci, selon la revendication 1, destiné à être utilisé à des fins de diagnostic.

7. Protéine CC10 des cellules Clara ou dérivé actif de celle-ci, selon la revendication 1, destiné à être utilisé pour déterminer le statut immun de l'organisme.
